# EUROPEAN PATENT SPECIFICATION

(11) **EP 2 523 134 B1**
(45) Date of publication and mention of the grant of the patent: **02.09.2020**
(21) Application number: 11166021.3
(22) Date of filing: 13.05.2011
(51) Int. Cl.: G16H 40/60

(54) **Methods and devices for managing sets of configuration parameters for laboratory management systems**
Verfahren und Vorrichtungen zum Verwalten von Konfigurationsparametersätzen für Laborverwaltungssysteme
Procédés et dispositifs de gestion des ensembles de paramètres de configuration pour systèmes de gestion de laboratoire

(43) Date of publication of application: 14.11.2012
(73) Proprietor: SYSMEX CORPORATION, Kobe-shi, Hyogo 651-0073 (JP)
(72) Inventor: Burbulla, Juergen, 22848 Norderstedt (DE); Kaulmann, Oliver, 1560 Hoeilaart (BE)
(74) Representative: Hoffmann Eitle

(56) References cited:
- WO-A2-2007/076970
- US-B1- 7 089 530

## Description

### [Technical field]

The present invention relates to method and devices for managing a plurality of sets of configuration parameters for use in laboratory management systems. These systems may include configurable analyzers, computer-implemented data management systems for controlling the data produced by the analyzers, etc. The invention also relates to computer programs configured for that purpose.

### [Background]

In the context of managing laboratory equipments and the configuration of these equipments, there is a need to configure in a different manner the equipments belonging to different users or group of users.

Laboratory data management apparatuses, such as work area managers (WAM) or laboratory information systems (LIS), are known in the art. For instance, document US 2007/0027648 describes a clinical testing information processing apparatus. Such laboratory data management apparatuses may be connected to analyzers to process the data outputted from the analyzers, i.e. to process the outputted analysis results. An analyzer may for instance be used to automatically analyze specimens such as blood and urine of a patient. An analyzer may for instance be a blood cell counting apparatus. Furthermore, an analyzer may for instance be located in a hospital. Client computers may be provided to access the analysis results outputted by an analyzer.

It is desirable to provide methods and devices to improve the handling and configuration of the laboratory management systems, while taking into account the need for computational efficiency, flexibility of operation and ease of use, and the need to reduce the costs of the network infrastructure and the operational cost.

### [Summary]

To meet or at least partially meet the above-mentioned goals, such methods and devices are defined in the independent claims. Advantageous embodiments are defined in the dependent claims.

The invention discloses a method for managing a plurality of sets of configuration parameters for laboratory management systems includes an inputting procedure, a creating procedure, and a modifying procedure. The inputting procedure includes being inputted with a first set of configuration parameter values. The first set is here referred to as parent set. The creating procedure, referred to here as child set creating procedure, includes creating at least one second set of configuration parameter values based on the parent set. Each second set of configuration parameter values is referred to here as child set. Creating a child set based on the parent set includes causing the child set to inherit all the configuration parameter values of the parent set, and, thereafter, if a child set's configuration parameter value inherited from the parent set is modified, the child set's configuration parameter whose value has been modified is regarded as a child-specific parameter. The modifying procedure includes modifying a configuration parameter value of the parent set, wherein the modification causes the value of the corresponding configuration parameter of a child set to be modified if the configuration parameter is not a child-specific parameter.

The method enables to efficiently and conveniently manage, i.e. create and maintain, a plurality of sets of configuration parameters and their values in the sense that it is at the same time possible to customize the configuration settings for different users or groups of users without the burden of having to repeatedly modify the same configuration parameter value for the different users or groups of users, at the risk of causing configuration errors. This is achieved by the conditional propagation to child sets of modifications made centrally to the parent set.

The method may be a computer-implemented method. In particular, a user interface may be provided for inputting the configuration parameter values of the parent set, for causing the computer to create a particular child set, and for deciding which of the configuration parameter values that the child set inherited from the parent set shall be a child-specific parameter, if any. Furthermore, the user interface may be provided with means to decide which of the configuration parameter values of the parent set is to be modified and how, so as to trigger the modifying procedure. The operation of the modifying procedure depends on whether the modified configuration parameter is a child-specific parameter.

A set of configuration parameters, for use in a laboratory management system, is a set of parameter values assigned to a number of parameters for use for configuring laboratory equipments and associated data management devices. For instance, a computer program or a group of computer programs constituting a software application may be configurable in the sense that, for instance, the value assigned to a particular configuration parameter may lead one user interface feature to be activated or deactivated respectively. For instance, depending on whether the value of a configuration parameter is equal to "true" or "false", one user interface feature may be activated or deactivated respectively.

Being inputted with configuration parameter values may include for instance receiving or obtaining a computer-readable file or data stream including a list of configuration parameter values, each corresponding to a configuration parameter. Being inputted with configuration parameter values may also mean receiving keyboard inputs representing a list of configuration parameter values, each corresponding to a configuration parameter.

The creating procedure may be triggered by a user activating a particular command of a supporting computer-implemented user interface, after for instance having inserted the name of the child set to be created from a parent set. The supporting computer-implemented user interface may also offer the opportunity to the user to select the configuration parameter value(s) inherited from the parent set that should be modified for a particular child set and how, i.e. to decide which configuration parameter(s) should become child-specific parameter(s), if any. The modifying procedure may also be triggered by a user activating a user command in a supporting computer-implemented user interface to decide which configuration parameter of the parent set should be modified, and with which value.

In one embodiment, the method further includes, before the inputting procedure, a further creating procedure, referred to here as parent set creating procedure, for creating the parent set. That is, a specific procedure may be provided to create the configuration parameter values of the parent set, and these configuration parameter values may then be provided as input to the inputting procedure.

In one embodiment, the modifying procedure for modifying a configuration parameter value of the parent set includes the following steps, for each child set: (i) if it is determined that the configuration parameter is not a child-specific parameter for a child set, the modification causes the value of the corresponding configuration parameter of the child set to be modified; and (ii) if it is determined that the configuration parameter is a child-specific parameter for a child set and if it is further determined, by obtaining or receiving an input from a user, that the child-specific parameter is to be overwritten, the modification causes the value of the corresponding configuration parameter of the child set to be modified. This embodiment enables to take into account a user input regarding whether a child-specific parameter should be overwritten when the corresponding configuration parameter in the parent set is modified.

In one embodiment, the configuration parameter values are values relating to the configuration settings of laboratory systems. A configuration parameter value may for instance be a Boolean value, a value among a finite number of possibilities, or a value among an infinite number of possibilities.

In one embodiment, the configuration parameter values are values relating to at least one of (a) how data is processed prior to being inputted into devices of a laboratory; (b) how data flows through devices of the laboratory; (c) how data is processed after being outputted from devices of the laboratory; (d) how devices, such as for instance analyzers, sorters and other laboratory equipments, are configured in the laboratory; (e) how user interfaces are displayed on computers in the laboratory; and (f) the access rights to some functions on computers in the laboratory. Processing data outputted from devices of the laboratory may notably include computer-implemented procedures such as performing measurement on the results, checking the validity of the results by applying certain rules on the results, deciding what to do with the results, and releasing the results for use by other equipments or by a physician or the like. In one embodiment, the configuration parameter values may be values relating to the workflow of processing of the resulting signals and data outputted by the laboratory equipments, so that the parameter values may represent how the results of the technical operations taking place in the laboratory equipments are processed.

In one embodiment, each set of configuration parameter values is used to configure a software application. A parent set and all of its child sets may each be used to configure a software application and these software applications may share the same source code.

In one embodiment, each set of configuration parameter values includes more than one hundred configuration parameter values.

In one embodiment, a device is provided for managing a plurality of sets of configuration parameters for laboratory management systems. The device includes an inputter, a creator, referred to here as child set creator, and a modifier. The inputter is configured for being inputted with a first set of configuration parameter values, the first set being referred to here as parent set. The child set creator is configured for creating at least one second set of configuration parameter values based on the parent set, wherein each second set is referred to here as child set. Creating a child set based on the parent set includes causing the child set to inherit all the configuration parameter values of the parent set, and, thereafter, if a child set's configuration parameter value inherited from the parent set is modified, the configuration parameter whose value has been modified is regarded as a child-specific parameter. The modifier is configured for modifying a configuration parameter value of the parent set, wherein the modification causes the value of the corresponding configuration parameter of a child set to be modified if the configuration parameter is not a child-specific parameter.

In one embodiment, a computer program includes computer-readable instructions configured, when executed on a computer, to cause the computer to carry out a method according to one of the above-described embodiments. Document US7089530 B1 discloses a control configuration system and represents the closest prior art.

### [Brief description of the drawings]

Embodiments of the present invention shall now be described, in conjunction with the appended figures, in which:
Fig. 1 is a flowchart of a method in one embodiment of the invention;
Fig. 2 is a flowchart of a method in one embodiment of the invention, schematically illustrating the repetition of the modifying procedure;
Fig. 3 is a flowchart of a method in one embodiment of the invention, including a parent set creating procedure;
Figs. 4 and 5 are flowcharts illustrating steps of modifying procedures in two embodiments of the invention;
Fig. 6 schematically illustrates the creation of child sets and the bundling of the child sets with some source code to form software applications in one embodiment of the invention; and
Figs. 7 and 8 schematically illustrate devices in two embodiments of the invention.

### [Detailed description]

The present invention shall now be described in conjunction with specific embodiments. These specific embodiments serve to provide the skilled person with a better understanding, but are not intended to in any way restrict the scope of the invention, which is defined by the appended claims.

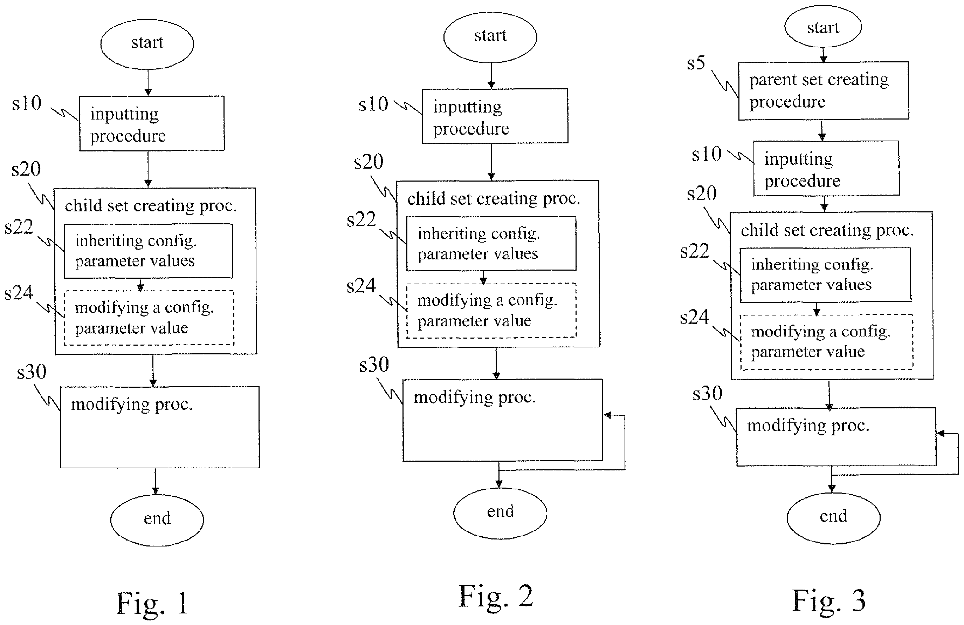

Fig. 1 is a flowchart of a method in one embodiment of the invention. In an inputting procedure s10, a first set of configuration parameter values are inputted to form the parent set. Inputting the parent set may be performed through the transfer of a data file or stream including the configuration parameter values of the parent set, or may involve entering by a user, and receiving by the inputting procedure s10, data corresponding to the configuration parameter values of the parent set.

The actual structure of the configuration parameters, i.e. the number of configuration parameters, their type if any, and the values that the configuration parameters may take, is not in itself part of the inputting procedure s10 but rather constitutes information common to the parent set and its child sets. During the operation of the method in embodiments of the invention, the actual structure of the configuration parameters does not change. That is, no configuration parameter is added or deleted, but the value of configuration parameters may be modified. Changing the actual structure of the configuration parameters may be viewed as a task taking place at another level than the level at which the method in embodiments of the invention takes place. In an exemplary context where the invention may be put into practice, changing the actual structure of the configuration parameters may be a task performed by software developers when developing the code source for a software application, whereas the method in embodiments of the invention, including the step of modifying the value of the configuration parameters of the parent set and propagating or not these modifications to each of the child sets, may be performed when creating and maintaining the configuration of different instances of the software application for different users or groups of users.

Then, after the inputting procedure s10, the child set creating procedure s20 is carried out for creating one or more child sets based on the parent set. The child set creating procedure s20 includes, for each child set, causing s22 the child set to inherit all the configuration parameter values of the parent set. Then, a configuration parameter value inherited by the child set from the parent set may be modified s24 in the child set. If so, this configuration parameter for which the value has been modified then becomes what is regarded as a child-specific parameter.

The step s24 of modifying a configuration parameter value of a child set is illustrated in Fig. 1, as well as in Figs. 2 and 3, using a dashed box. This is to indicate that a child set may be created by inheriting all the configuration parameter values of the parent set without any modification to the child set's configuration parameter values. In that case, upon creation, there is no child-specific parameter for that child set. This however does not prejudice the possibility to later create a child-specific parameter by modifying a configuration parameter of the child set. In one embodiment, at least one configuration parameter value of each of most of the child sets is modified s24 upon creation of the child set. In one embodiment, at least one configuration parameter value of each of all the child sets is modified s24 upon creation of the child set.

The modifying procedure s30 includes modifying a configuration parameter value of the parent set, wherein the modification causes the value of the corresponding configuration parameter of a child set to be modified if the configuration parameter of the child set is not a child-specific parameter. The modifying procedure s30 may include, as a trigger, receiving a user input, for instance in a user interface, indicating that a configuration parameter value of the parent set shall be modified and how the value shall be modified. Whether a modification made to a parent set's configuration parameter value is propagated to its child sets is determined on a per-child-set basis. Namely, as a result of the per-child-set determination, a modification may be propagated to all child sets, to one or some of the child sets, or to none of the child sets.

Although not illustrated in Fig. 1, the modification of a configuration parameter value of a child set may be performed at any time so as to create further child-specific parameters. In other words, further occurrences of step s24 may be performed after s30. This may occur if a user decides to further customize the configuration for the child set.

Whether a configuration parameter of a particular child set is regarded at one point in time as a child-specific parameter is, in a first embodiment, recorded, for instance using a flag or the like. This is a way to remember that the configuration parameter is a child-specific parameter, i.e. that the configuration parameter has been specifically modified for the child set.

Alternatively, in a second embodiment, there is no particular flag or the like indicating that a configuration parameter is regarded as a child-specific parameter. In this alternative embodiment, a configuration parameter may be determined to be child-specific if the configuration parameter value assigned to the configuration parameter for the child set and the configuration parameter value assigned to the parent set for the configuration parameter are different.

These two embodiments may have different implications as to what is considered a child-specific parameter. In the first embodiment where a flag or the like is used to remember the child-specific character of the configuration parameters, if, after modification of the value of a configuration parameter of the child set to create a child-specific parameter, the value of the corresponding configuration parameter of the parent set is modified and, at the occasion of this modification, the value becomes equal to the value of the configuration parameter of the child set, then the configuration parameter of the child set does not lose its child-specific character. This means that, when the value of the corresponding configuration parameters of the parent and child set are equal and when the value of the configuration parameter of the parent set is modified to another value, the value of the corresponding configuration parameter of a child set is not caused to be modified. No persistence of an alignment of values has been created between the parent and child set. The child-specific character rather persists in the process.

In the second embodiment, if, after creating a child set having a child-specific parameter and after modifying the value of the corresponding configuration parameter of the parent set so that the value of this configuration parameter of the parent set becomes equal to the value of the configuration parameter of the child set, the configuration parameter of the child set loses the child-specific character and will therefore be aligned later on if there is a further modification to the configuration parameter of the parent set. A persistence of an alignment of values has been created between the parent and child set.

This may be illustrated by the following example, with four configuration parameters P1, P2, P3 and P4 (a set will however typically have many more values). At a time t = 0, the parent set's configuration parameters values are respectively { P1=1, P2=true, P3=3, P4=x } and a child set is created s20 with configuration parameters values { P1=1, P2=true, P3=5, P4=x } wherein P3 is therefore a child-specific value for the child set. This is illustrated by table 0.

**Table 0.**

| | | | | | |
|---|---|---|---|---|---|
| Time t = 0 | Parent set: | P1=1 | P2=true | P3=3 | P4=x |
| | Child set: | P1=1 | P2=true | P3=5 | P4=x |

At time t = 1, the value of the parent set's P3 configuration parameter is changed from P3=3 to P3=5. At that stage, according to the modifying procedure s30, it may be determined whether the child set's P3 configuration parameter is a child-specific parameter for the child set so as to decide whether to modify the value of the child set's P3 configuration parameter or not. However, since in any case, the modification of the value of the parent set's P3 configuration parameter leads to an alignment of the values of the parent set's and child set's P3 configuration parameter, this determination does not really matter and may be skipped. This is illustrated by table 1.

**Table 1.**

| | | | | | |
|---|---|---|---|---|---|
| Time t = 1 | Parent set: | P1=1 | P2=true | P3=5 | P4=x |
| | Child set: | P1=1 | P2=true | P3=5 | P4=x |

At time t = 2, the value of the parent set's P3 configuration parameter is further changed from P3=5 to P3=7. At that stage, according to the modifying procedure s30, it is determined whether the child set's P3 configuration parameter is a child-specific parameter for the child set. According to the first embodiment, since whether the child set's P3 configuration parameter is a child-specific parameter for the child set is determined based on a flag or the like, the child-specific character persists and the value of the child set's P3 configuration parameter is not modified at time t = 2. This is illustrated by table 2-1.

**Table 2-1.**

| | | | | | |
|---|---|---|---|---|---|
| Time t = 2 (1st embodiment) | Parent set: | P1=1 | P2=true | P3=7 | P4=x |
| | Child set: | P1=1 | P2=true | P3=5 | P4=x |

According to the second embodiment, since whether the child set's P3 configuration parameter is a child-specific parameter for the child set is determined based on comparing the values of the parent set's and child set's P3 configuration parameter, the child-specific character has been lost in time t = 1 and the value of the child set's P3 configuration parameter is modified at time t = 2. This is illustrated by table 2-2.

**Table 2-2.**

| | | | | | |
|---|---|---|---|---|---|
| Time t = 2 (2nd embodiment) | Parent set: | P1=1 | P2=true | P3=7 | P4=x |
| | Child set: | P1=1 | P2=true | P3=7 | P4=x |

According to a third embodiment, a user input at time t = 2 is sought and obtained regarding whether the child set's P3 configuration parameter should be modified to the value P3=5 or P3=7. This has the advantage of leaving the user to decide in such a borderline case whether the child set's P3 configuration parameter should keep its child-specific character or not.

In the example illustrated by Tables 0, 1, 2-1 and 2-2, and more generally in any embodiment of the invention, the values of the configuration parameters P1, P2, P3 and P4 for a parent set and any child set may be stored in a data storage medium. The data storage medium may be any type of medium such as for instance a hard disk, a ROM or RAM memory, a flash memory, etc.

In view of the previous paragraph, in one embodiment, the inputting procedure s10 for being inputted with the parent set may include (i) initially obtaining the parent set's configuration parameter values for instance through user inputs on a visual user interface, and (ii) storing all the obtained parent set's configuration parameter values in a specific location of the data storage medium, along with identifiers indicating to which configuration parameter each stored value corresponds.

The child set creating procedure s20 for creating at least one child set may likewise include storing all the child set's configuration parameter values in another specific location of the data storage medium, along with identifiers indicating to which configuration parameter each stored value for the child set corresponds. In the child set creating procedure s20, causing s22 the child set to inherit all the configuration parameter values of the parent set may include (i) retrieving the parent set's configuration parameter values from the specific location of the data storage medium where these values are stored, (ii) temporarily copying these values in a volatile memory, and then (iii) storing the copied values in the above-mentioned other specific location of the data storage medium, along with the identifiers indicating to which configuration parameter each stored value for the child set corresponds. In the child set creating procedure s20, modifying s24 a configuration parameter value inherited from the parent set to form a child-specific parameter may include (i) obtaining the modified child set's configuration parameter values through user inputs on a visual user interface, and (ii) modifying the obtained corresponding configuration parameter value stored for the child set in the data storage medium and optionally storing a corresponding Boolean flag indicating that the child set configuration parameter became a child-specific parameter.

The modifying procedure s30 for modifying a configuration parameter value of the parent set may include (i) obtaining the modified parent set's configuration parameter values through user inputs on a visual user interface, (ii) modifying the corresponding parent set configuration parameter value stored for the parent set in the data storage medium, (iii)(a) retrieving from the data storage medium the value of a flag to determine whether the corresponding configuration parameter of the child set is child-specific or (iii)(b) retrieving from the data storage medium the value of the corresponding configuration parameter of the child set to determine whether the corresponding configuration parameter of the child set is child-specific by comparing the parent set configuration parameter value and the child set configuration parameter value, and (iv) modifying the value of the corresponding configuration parameter of a child set in the data storage medium if the configuration parameter is determined not to be a child-specific parameter.

Fig. 2 is a flowchart of method in one embodiment of the invention, which differs from the embodiment illustrated in Fig. 1 in that Fig. 2 additionally illustrates repeating the modifying procedure s30. Namely, not just one but a plurality of configuration parameters of the parent set may be subject to modification. These modifications cause the value of the corresponding configuration parameters of the child sets to be modified under certain conditions. In particular, if a configuration parameter is not a child-specific parameter for a child set, the modification to the parent set causes the value of the corresponding configuration parameter of the child set to be modified as well. In other words, this corresponds to the conditional propagation of a modification to the parent set to its child sets.

Fig. 3 is a flowchart of a method in one embodiment of the invention, which differs from the method illustrated in Fig. 2 in that, in Fig. 3, a further step of creating s5 a parent set is illustrated. This parent set creating procedure s5 may for instance include opening dialog boxes and more generally using a computer-implemented user interface to assign values to configuration parameters to create the data for forming parent set. The data is then used as input for the inputting procedure s10 as illustrated in Fig. 3.

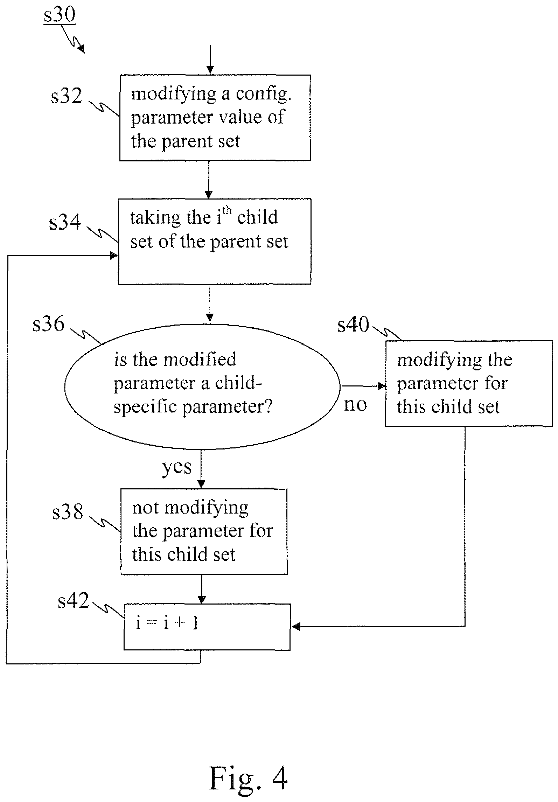

Fig. 4 is a flowchart illustrating steps of a modifying procedure s30 which may belong to the method illustrated in any one of Figs. 1 to 3. After a configuration parameter value of the parent set is modified s32, each child set is considered, either in sequence (as illustrated in Fig. 4) or in parallel (not illustrated in Fig. 4, but also possible). Fig. 4 illustrates a step of taking s34 the i^{th} child set of the parent set and a step s42 of incrementing i ("i=i+1"), so as to illustrate an iteration being performed on the child set of the parent set. For the sake of simplicity, Fig. 4 does not illustrate the iteration's initialization, for instance consisting in setting i = 1, and its termination, for instance consisting in ending the iteration when it is determined i > n, with n being the number of child sets.

For each child set, the method includes determining s36 whether the modified configuration parameter is, for the considered child set, a child-specific parameter. If so (Fig. 4, "yes" after step s36), the value of the corresponding configuration parameter of the child set is not modified s38. Otherwise (Fig. 4, "no" after step s36), the value of the corresponding configuration parameter of the child set is modified s40.

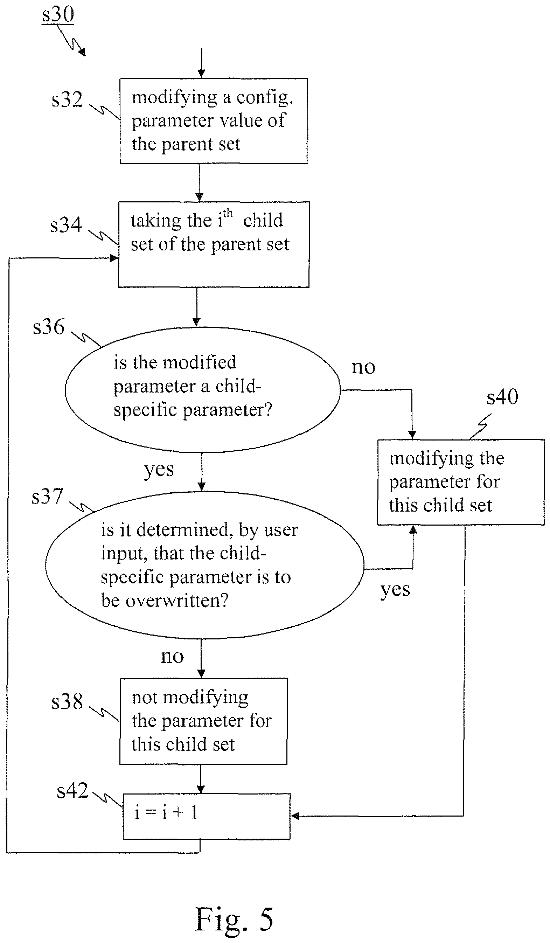

Fig. 5 is a flowchart of steps of the modifying procedure s30 in another embodiment of the invention.

After step s36 of determining whether the modified configuration parameter is a child-specific parameter and if the determination is positive (Fig. 5, "yes" after step s36), it is determined s37, by receiving or obtaining a user input (this may for instance be implemented by alerting the user that he or she is attempting to modify a child-specific parameter), whether the child-specific parameter is to be overwritten. If an instruction is received or obtained from the user that the child-specific parameter is to be overwritten (Fig. 5, "yes" after step s37), the configuration parameter of the child set is modified s40. If, however, the user, through the input provided to the computer-implemented method, indicates that the child-specific parameter must not be overwritten (Fig. 5, "no" after step s37), the configuration parameter of this child set is not modified s38.

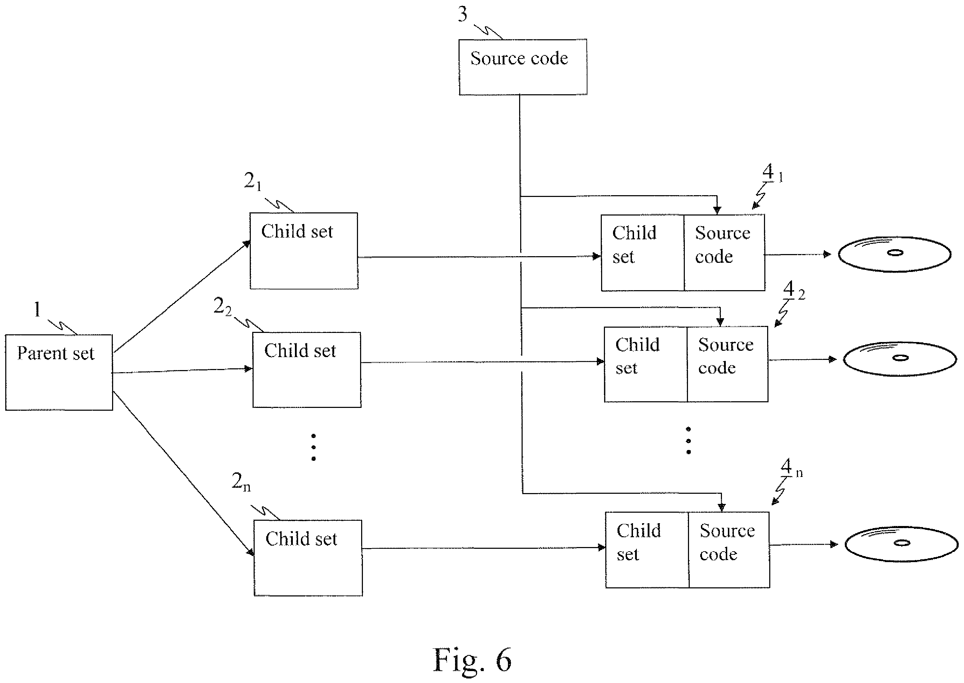

Fig. 6 is a schematic representation of the creation of child sets 2₁, 2₂, ..., 2ₙ from a parent set 1, and then the bundling of the child sets with source code 3 to form software applications for 4₁, 4₂, ..., 4ₙ. These software applications may be delivered to remote locations for instance through their storage on CD-ROMs (as illustrated on Fig. 6) or by transmission through other means, such as through a network (not illustrated on Fig. 6) for uploading the configuration on the premises (such as for instance a hospital or a laboratory) where the software applications are to be used.

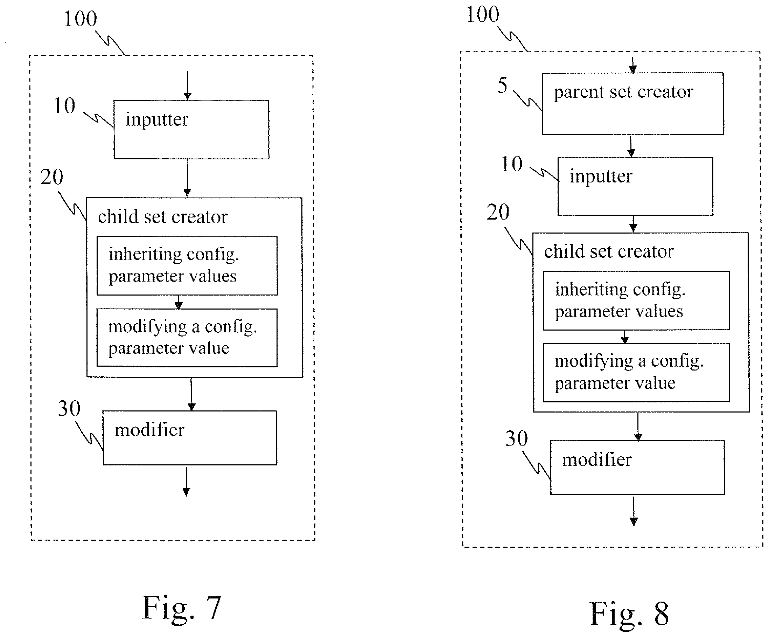

Fig. 7 schematically illustrates a device 100 in one embodiment of the invention. The device 100 includes an inputter 10, a child set creator 20 and a modifier 30.

The device 100 is used for managing a plurality of sets of configuration parameters for laboratory management systems. The inputter 10 is configured for being inputted with a parent set. The child set creator 20 is configured for creating at least one child set. Creating a child set based on a parent set includes, in the child set creator 20, causing the child set to inherit all the configuration parameter values of the parent set, and optionally modifying at least one of the configuration parameter values inherited from the parent set. If an inherited configuration parameter is modified this creates a child-specific parameter. The modifier 30 is configured for modifying a configuration parameter value of the parent set, wherein this modification may be triggered by a user input made in a user interface. This modification causes the value of the corresponding configuration parameter of the child set to be modified if the configuration parameter, for this child set, is not a child-specific parameter.

Fig. 8 schematically illustrates a device according to another embodiment of the invention. Compared to the device schematically illustrated in Fig. 7, the device schematically illustrated in Fig. 8 further includes a parent set creator 5 configured for creating the parent set.

A parent set (the master set) and a plurality of child sets may be maintained in such a manner that each of the child sets is intended to be used for a particular type of users or groups of users. For instance, a first child set is used for the European regional market and a second child set is used for the African regional market. The sets, corresponding to a particular version instance of the software application, are then shipped or updated to their corresponding market with the same source code. The use of the distinct sets ensures that the source code translates into distinctly configured laboratory management software applications in the different markets, this being achieved in an efficient and convenient manner. While a single parent set is used, different functions are provided to different users or groups of users (such as different regions, different countries, different markets within a country, different customer groups, different lab solutions, different training and skill levels of the deployment staff and consultants, etc.), making it particularly convenient to develop and maintain the software applications on a global perspective. This also reduces the risk of human errors when configuring and maintaining the software applications, which is particularly important in the context of laboratory management software applications.

The physical entities according to the invention or according to embodiments thereof, including the device 100, may comprise or store computer programs including instructions such that, when the computer programs are executed on the physical entities, steps and procedures according to embodiments of the invention are carried out. The invention also relates to such computer programs for carrying out methods according to the invention, and to any computer-readable medium storing the computer programs for carrying out methods according to the invention.

Where the terms "inputter", "child set creator", "modifier" and "parent set creator" are used herewith, no restriction is made regarding how distributed these elements may be and regarding how gathered elements may be. That is, the constituent elements of an inputter, child set creator, modifier and parent set creator may be distributed in different software or hardware components or devices for bringing about the intended function. A plurality of distinct elements may also be gathered for providing the intended functionalities.

Any one of the above-referred elements of an inputter, child set creator, modifier and parent set creator, may be implemented in hardware, software, field-programmable gate array (FPGA), application-specific integrated circuit (ASICs), firmware or the like.

In further embodiments of the invention, any one of the above-mentioned and/or claimed inputter, child set creator, modifier and parent set creator is replaced by an inputting unit, a child set creating unit, a modifying unit and a parent set creating unit respectively, or inputting means, child set creating means, modifying means and parent set creating means respectively, for performing the functions of inputter, child set creator, modifier and parent set creator.

In further embodiments of the invention, any one of the above-described procedures or steps may be implemented using computer-readable instructions, for example in the form of computer-understandable procedures, methods or the like, in any kind of computer languages, and/or in the form of embedded software on firmware, integrated circuits or the like.

Although the present invention has been described on the basis of detailed examples, the detailed examples only serve to provide the skilled person with a better understanding, and are not intended to limit the scope of the invention. The scope of the invention is much rather defined by the appended claims.

## Claims

1. Computer-implemented method for managing a plurality of sets of configuration parameters for laboratory management systems including
an inputting procedure (s10) for being inputted with a first set of configuration parameter values, the first set being referred to here as parent set;
a creating procedure, referred to here as child set creating procedure (s20), for creating at least one second set of configuration parameter values based on the parent set, each second set being referred to here as child set, wherein creating a child set based on the parent set includes:
causing (s22) the child set to inherit all the configuration parameter values of the parent set,
wherein, after the step of causing (s22), if a child set's configuration parameter value inherited from the parent set is modified (s24), the child set's configuration parameter whose value has been modified is regarded as a child-specific parameter; and
a modifying procedure (s30) for modifying a configuration parameter value of the parent set, wherein the modification causes the value of the corresponding configuration parameter of a child set to be modified if the configuration parameter is not a child-specific parameter.

2. Method of claim 1, wherein the child set creating procedure (s20) further includes modifying (s24) at least one of the configuration parameter values inherited from the parent set, each of the at least one configuration parameter whose value has been modified being then regarded as child-specific parameter.

3. Method of claim 1 or 2, further including, before the inputting procedure (s10), a further creating procedure, referred to here as parent set creating procedure (s5), for creating the parent set.

4. Method according to any one of the preceding claims, wherein the modifying procedure (s30) for modifying a configuration parameter value of the parent set includes
if it is determined (s36) that, for a child set, the corresponding configuration parameter is not a child-specific parameter, the modification causes the value of the corresponding configuration parameter of the child set to be modified (s40); and
if it is determined (s36) that, for a child set, the corresponding configuration parameter is a child-specific parameter and if it is determined (s37), by obtaining or receiving an input from a user, that the child-specific parameter is to be overwritten, the modification causes the value of the corresponding configuration parameter of the child set to be modified (s40).

5. Method according to any one of the preceding claims, wherein the configuration parameter values are values relating to the configuration settings of laboratory systems.

6. Method of claim 5, wherein the configuration parameter values are values relating to at least one of:
how data is processed prior to being inputted into devices of a laboratory;
how data flows through devices of the laboratory;
how data is processed after being outputted from devices of the laboratory;
how devices are configured in the laboratory;
how user interfaces are displayed on computers in the laboratory; and
the access rights to some functions on computers in the laboratory.

7. Method according to any one of the preceding claims, each set of configuration parameter values is used to configure a software application.

8. Method of claim 7, wherein a parent set (1) and all its child sets (2₁, 2₂, ..., 2ₙ) are each used to configure a software application (4₁, 4₂, ..., 4ₙ) and these software applications share the same source code (3).

9. Method according to any one of the preceding claims, each set of configuration parameter values includes more than one hundred configuration parameter values.

10. Device (100) for managing a plurality of sets of configuration parameters for laboratory management systems including
an inputter (10) configured for being inputted with a first set of configuration parameter values, the first set being referred to here as parent set;
a creator, referred to here as child set creator (20), configured for creating at least one second set of configuration parameter values based on the parent set, each second set being referred to here as child set, wherein creating a child set based on the parent set includes:
causing the child set to inherit all the configuration parameter values of the parent set,
wherein, thereafter, if a child set's configuration parameter value inherited from the parent set is modified, the child set's configuration parameter value is regarded as a child-specific parameter; and
a modifier (30) configured for modifying a configuration parameter value of the parent set, wherein the modification causes the value of the corresponding configuration parameter of a child set to be modified if the configuration parameter is not a child-specific parameter.

11. Device of claim 10, wherein the child set creator (20) is further configured to modify at least one of the configuration parameter values inherited from the parent set, each of the at least one configuration parameter whose value has been modified being then regarded as child-specific parameter.

12. Device (100) of claim 10 or 11, further including a further creator, referred to here as parent set creator (5), configured for creating the parent set.

13. Device (100) according to any one of claims 10 to 12, wherein the modifier (30) configured for modifying a configuration parameter value of the parent set is configured for,
if the modifier (30) determines that, for a child set, the corresponding configuration parameter is not a child-specific parameter, causing the value of the corresponding configuration parameter of a child set to be modified; and
if the modifier (30) determines that, for a child set, the corresponding configuration parameter is a child-specific parameter and the modifier (30) determines, by obtaining or receiving an input from a user, that the child-specific parameter is to be overwritten, causing the value of the corresponding configuration parameter of the child set to be modified.

14. Device (100) according to any one of claims 10 to 13, wherein the configuration parameter values are values relating to the configuration settings of laboratory systems.

15. Device (100) of claim 14, wherein the configuration parameter values are values relating to at least one of:
how data is processed prior to being inputted into devices of a laboratory;
how data flows through devices of the laboratory;
how data is processed after being outputted from devices of the laboratory;
how devices are configured in the laboratory;
how user interfaces are displayed on computers in the laboratory; and
the access rights to some functions on computers in the laboratory.

16. Computer program including computer-readable instructions configured, when executed on a computer, to cause the computer to carry out the method according to any one of claims 1 to 9.

## Patentansprüche

1. Computerimplementiertes Verfahren zum Verwalten einer Vielzahl von Konfigurationsparametersätzen für Laborverwaltungssysteme, beinhaltend
einen Eingabevorgang (s10), welcher mit einem ersten Satz von Konfigurationsparameterwerten eingegeben werden soll, wobei der erste Satz hier als übergeordneter Satz bezeichnet wird;
einen Erstellungsvorgang, welcher hier als Erstellungsvorgang eines untergeordneten Satzes (s20) bezeichnet wird, zum Erstellen zumindest eines zweiten Satzes von Konfigurationsparameterwerten auf Basis des übergeordneten Satzes, wobei jeder zweite Satz hier als untergeordneter Satz bezeichnet wird, wobei Erstellen eines untergeordneten Satzes auf Basis des übergeordneten Satzes beinhaltet:
Veranlassen (s22) des untergeordneten Satzes, alle Konfigurationsparameterwerte des übergeordneten Satzes zu übernehmen;
wobei nach dem Schritt des Veranlassens (s22), wenn ein von dem übergeordneten Satz übernommener Konfigurationsparameterwert eines untergeordneten Satzes modifiziert wird (s24), der Konfigurationsparameter des untergeordneten Satzes, dessen Wert modifiziert worden ist, als ein spezifischer Parameter des untergeordneten Satzes betrachtet wird; und
einen Modifizierungsvorgang (s30) zum Modifizieren eines Konfigurationsparameterwerts des übergeordneten Satzes, wobei die Modifizierung veranlasst, dass der Wert des zugehörigen Konfigurationsparameters eines untergeordneten Satzes modifiziert wird, wenn der Konfigurationsparameter kein spezifischer Parameter des untergeordneten Satzes ist.

2. Verfahren nach Anspruch 1, wobei der Erstellungsvorgang eines untergeordneten Satzes (s20) weiter Modifizieren (s24) zumindest eines der von dem übergeordneten Satz übernommenen Konfigurationsparameterwerte beinhaltet, wobei jeder von dem zumindest einen Konfigurationsparameter, dessen Wert modifiziert wurde, dann als spezifischer Parameter des untergeordneten Satzes betrachtet wird.

3. Verfahren nach Anspruch 1 oder 2, vor dem Eingabevorgang (s10) weiter einen weiteren Erstellungsvorgang, welcher hier als Erstellungsvorgang eines übergeordneten Satzes (s5) bezeichnet wird, zum Erstellen des übergeordneten Satzes beinhaltend.

4. Verfahren nach einem der vorstehenden Ansprüche, wobei der Modifizierungsvorgang (s30) zum Modifizieren eines Konfigurationsparameterwerts des übergeordneten Satzes beinhaltet:
falls bestimmt wird (s36), dass für einen untergeordneten Satz der zugehörige Konfigurationsparameter kein spezifischer Parameter des untergeordneten Satzes ist, veranlasst die Modifizierung, dass der Wert des zugehörigen Konfigurationsparameters des untergeordneten Satzes modifiziert wird (s40); und
falls bestimmt wird (s36), dass für einen untergeordneten Satz der zugehörige Konfigurationsparameter ein spezifischer Parameter des untergeordneten Satzes ist und falls durch Erhalten oder Empfangen einer Eingabe von einem Benutzer bestimmt wird (s37), dass der spezifische Parameter des untergeordneten Satzes überschrieben werden soll, veranlasst die Modifizierung, dass der Wert des zugehörigen Konfigurationsparameters des untergeordneten Satzes modifiziert wird (s40).

5. Verfahren nach einem der vorstehenden Ansprüche, wobei die Konfigurationsparameterwerte Werte sind, welche sich auf die Konfigurationseinstellungen von Laborsystemen beziehen.

6. Verfahren nach Anspruch 5, wobei die Konfigurationsparameterwerte Werte sind, welche sich zumindest auf eines der folgenden beziehen:
wie Daten verarbeitet werden, bevor sie in Vorrichtungen eines Labors eingegeben werden;
wie Daten durch Vorrichtungen des Labors fließen;
wie Daten verarbeitet werden, nachdem sie von Vorrichtungen des Labors ausgegeben werden;
wie Vorrichtungen in dem Labor konfiguriert werden;
wie Benutzeroberflächen auf Computern in dem Labor angezeigt werden; und
die Zugriffsrechte auf einige Funktionen auf Computern in dem Labor.

7. Verfahren nach einem der vorstehenden Ansprüche, wobei jeder Satz von Konfigurationsparameterwerten verwendet wird, um eine Softwareanwendung zu konfigurieren.

8. Verfahren nach Anspruch 7, wobei ein übergeordneter Satz (1) und alle seine untergeordneten Sätze (2₁, 2₂, ..., 2ₙ) jeweils verwendet werden, um eine Softwareanwendung (4₁, 4₂, ..., 4ₙ) zu konfigurieren, und diese Softwareanwendungen einen gemeinsamen Sourcecode (3) aufweisen.

9. Verfahren nach einem der vorstehenden Ansprüche, wobei jeder Satz von Konfigurationsparameterwerten mehr als einhundert Konfigurationsparameterwerte beinhaltet.

10. Vorrichtung (100) zum Verwalten einer Vielzahl von Konfigurationsparametersätzen für Laborverwaltungssysteme, beinhaltend
ein Eingabegerät (10), welches konfiguriert ist, um mit einem ersten Satz von Konfigurationsparameterwerten eingegeben zu werden, wobei der erste Satz hier als übergeordneter Satz bezeichnet wird;
ein Erstellungsgerät, welches hier als Erstellungsgerät eines untergeordneten Satzes (20) bezeichnet wird, welches konfiguriert ist, um zumindest einen zweiten Satz von Konfigurationsparameterwerten auf Basis des übergeordneten Satzes zu erstellen, wobei jeder zweite Satz hier als untergeordneter Satz bezeichnet wird, wobei Erstellen eines untergeordneten Satzes auf Basis des übergeordneten Satzes beinhaltet:
Veranlassen des untergeordneten Satzes, alle Konfigurationsparameterwerte des übergeordneten Satzes zu übernehmen;
wobei danach, wenn ein von dem übergeordneten Satz übernommener Konfigurationsparameterwert eines untergeordneten Satzes modifiziert wird, der Konfigurationsparameterwert des untergeordneten Satzes als ein spezifischer Wert des untergeordneten Satzes betrachtet wird; und
ein Modifizierungsgerät (30), welches konfiguriert ist, um einen Konfigurationsparameterwert des übergeordneten Satzes zu modifizieren, wobei die Modifizierung veranlasst, dass der Wert des zugehörigen Konfigurationsparameters eines untergeordneten Satzes modifiziert wird, wenn der Konfigurationsparameter kein spezifischer Parameter des untergeordneten Satzes ist.

11. Vorrichtung nach Anspruch 10, wobei das Erstellungsgerät eines untergeordneten Satzes (20) weiter konfiguriert ist, um zumindest einen der von dem übergeordneten Satz übernommenen Konfigurationsparameterwerte zu modifizieren, wobei jeder von dem zumindest einen Konfigurationsparameter, dessen Wert modifiziert wurde, dann als spezifischer Parameter des untergeordneten Satzes betrachtet wird.

12. Vorrichtung (100) nach Anspruch 10 oder 11, weiter ein weiteres Erstellungsgerät, welches hier als Erstellungsgerät eines übergeordneten Satzes (5) bezeichnet wird, beinhaltend, welches konfiguriert ist, um den übergeordneten Satz zu erstellen.

13. Vorrichtung (100) nach einem der Ansprüche 10 bis 12, wobei das Modifizierungsgerät (30), welches konfiguriert ist, um einen Konfigurationsparameterwert des übergeordneten Satze zu modifizieren, konfiguriert ist, um:
falls das Modifizierungsgerät (30) bestimmt, dass für einen untergeordneten Satz der zugehörige Konfigurationsparameter kein spezifischer Parameter des untergeordneten Satzes ist, zu veranlassen, dass der Wert des zugehörigen Konfigurationsparameters eines untergeordneten Satzes modifiziert wird; und
falls das Modifizierungsgerät (30) bestimmt, dass für einen untergeordneten Satz der zugehörige Konfigurationsparameter ein spezifischer Parameter des untergeordneten Satzes ist und das Modifizierungsgerät (30) durch Erhalten oder Empfangen einer Eingabe von einem Benutzer bestimmt, dass der spezifische Parameter des untergeordneten Satzes überschrieben werden soll, zu veranlassen, dass der Wert des zugehörigen Konfigurationsparameters des untergeordneten Satzes modifiziert wird.

14. Vorrichtung (100) nach einem der Ansprüche 10 bis 13, wobei die Konfigurationsparameterwerte Werte sind, welche sich auf die Konfigurationseinstellungen von Laborsystemen beziehen.

15. Vorrichtung (100) nach Anspruch 14, wobei die Konfigurationsparameterwerte Werte sind, welche sich zumindest auf eines der folgenden beziehen:
wie Daten verarbeitet werden, bevor sie in Vorrichtungen eines Labor eingegeben werden;
wie Daten durch Vorrichtungen des Labors fließen;
wie Daten verarbeitet werden, nachdem sie von Vorrichtungen des Labors ausgegeben werden;
wie Vorrichtungen in dem Labor konfiguriert werden;
wie Benutzeroberflächen auf Computern in dem Labor angezeigt werden; und
die Zugriffsrechte auf einige Funktionen auf Computern in dem Labor.

16. Computerprogramm, beinhaltend computerlesbare Anweisungen, welche konfiguriert sind, wenn auf einem Computer ausgeführt, um den Computer zu veranlassen, das Verfahren nach einem der Ansprüche 1 bis 9 auszuführen.

## Revendications

1. Procédé mis en œuvre sur ordinateur pour la gestion d'une pluralité d'ensembles de paramètres de configuration pour systèmes de gestion de laboratoire incluant
une procédure d'entrée (s10) destinée à recevoir en tant qu'entrée un premier ensemble de valeurs de paramètre de configuration, le premier ensemble étant désigné ici comme un ensemble parent ;
une procédure de création, désignée ici comme une procédure de création d'ensemble enfant (s20), pour créer au moins un second ensemble de valeurs de paramètre de configuration sur la base de l'ensemble parent, chaque second ensemble étant désigné ici comme un ensemble enfant, dans lequel la création d'un ensemble enfant sur la base de l'ensemble parent inclut :
le fait d'amener (s22) l'ensemble enfant à hériter de toutes les valeurs de paramètre de configuration de l'ensemble parent,
dans lequel, après l'étape d'amenée (s22), si une valeur de paramètre de configuration d'un ensemble enfant héritée de l'ensemble parent est modifiée (s24), le paramètre de configuration de l'ensemble enfant dont la valeur a été modifiée est considéré comme un paramètre spécifique d'enfant ; et
une procédure de modification (s30) pour modifier une valeur de paramètre de configuration de l'ensemble parent, dans lequel la modification amène la valeur du paramètre de configuration correspondant d'un ensemble enfant à être modifiée si le paramètre de configuration n'est pas un paramètre spécifique d'enfant.

2. Procédé selon la revendication 1, dans lequel la procédure de création d'ensemble enfant (s20) inclut en outre la modification (s24) d'au moins l'une des valeurs de paramètre de configuration héritées de l'ensemble parent, chacun de l'au moins un paramètre de configuration dont la valeur a été modifiée étant alors considéré comme étant un paramètre spécifique d'enfant.

3. Procédé selon la revendication 1 ou 2, incluant en outre, avant la procédure d'entrée (s10), une procédure de création supplémentaire, désignée ici comme une procédure de création d'ensemble parent (s5), pour créer l'ensemble parent.

4. Procédé selon l'une quelconque des revendications précédentes, dans lequel la procédure de modification (s30) pour modifier une valeur de paramètre de configuration de l'ensemble parent inclut
s'il est déterminé (s36) que, pour un ensemble enfant, le paramètre de configuration correspondant n'est pas un paramètre spécifique d'enfant, la modification amène la valeur du paramètre de configuration correspondant de l'ensemble enfant à être modifiée (s40) ; et
s'il est déterminé (s36) que, pour un ensemble enfant, le paramètre de configuration correspondant est un paramètre spécifique d'enfant et s'il est déterminé (s37), par obtention ou réception d'une entrée à partir d'un utilisateur, que le paramètre spécifique d'enfant doit être écrasé, la modification amène la valeur du paramètre de configuration correspondant de l'ensemble enfant à être modifiée (s40).

5. Procédé selon l'une quelconque des revendications précédentes, dans lequel les valeurs de paramètre de configuration sont des valeurs relatives aux réglages de configuration de systèmes de laboratoire.

6. Procédé selon la revendication 5, dans lequel les valeurs de paramètre de configuration sont des valeurs relatives à au moins l'un de :
la manière dont les données sont traitées avant d'être entrées dans des dispositifs d'un laboratoire ;
la manière dont les données circulent à travers les dispositifs du laboratoire ;
la manière dont les données sont traitées après être sorties des dispositifs du laboratoire ;
la manière dont les dispositifs sont configurés dans le laboratoire ;
la manière dont les interfaces utilisateur sont affichées sur les ordinateurs dans le laboratoire ; et
les droits d'accès à certaines fonctions sur les ordinateurs du laboratoire.

7. Procédé selon l'une quelconque des revendications précédentes, chaque ensemble de valeurs de paramètre de configuration est utilisé pour configurer une application logicielle.

8. Procédé selon la revendication 7, dans lequel un ensemble parent (1) et tous ses ensembles enfants (2₁, 2₂, ..., 2ₙ) sont chacun utilisés pour configurer une application logicielle (4₁, 4₂, ..., 4ₙ) et ces applications logicielles partagent le même code source (3).

9. Procédé selon l'une quelconque des revendications précédentes, chaque ensemble de valeurs de paramètre de configuration inclut plus de cent valeurs de paramètre de configuration.

10. Dispositif (100) pour la gestion d'une pluralité d'ensembles de paramètres de configuration pour systèmes de gestion de laboratoire incluant
un dispositif d'entrée (10) configuré pour recevoir en tant qu'entrée un premier ensemble de valeurs de paramètre de configuration, le premier ensemble étant désigné ici comme un ensemble parent ;
un créateur, désigné ici comme un créateur d'ensemble enfant (20), configuré pour créer au moins un second ensemble de valeurs de paramètre de configuration sur la base de l'ensemble parent, chaque second ensemble étant désigné ici comme un ensemble enfant, dans lequel la création d'un ensemble enfant sur la base de l'ensemble parent inclut :
le fait d'amener l'ensemble enfant à hériter de toutes les valeurs de paramètre de configuration de l'ensemble parent,
dans lequel, ci-après, si une valeur de paramètre de configuration d'un ensemble enfant héritée de l'ensemble parent est modifiée, la valeur de paramètre de configuration d'un ensemble enfant est considérée comme un paramètre spécifique d'enfant ; et
un modificateur (30) configuré pour modifier une valeur de paramètre de configuration de l'ensemble parent, dans lequel la modification amène la valeur du paramètre de configuration correspondant d'un ensemble enfant à être modifiée si le paramètre de configuration n'est pas un paramètre spécifique d'enfant.

11. Dispositif selon la revendication 10, dans lequel le créateur d'ensemble enfant (20) est en outre configuré pour modifier au moins l'une des valeurs de paramètre de configuration héritées de l'ensemble parent, chacun de l'au moins un paramètre de configuration dont la valeur a été modifiée étant alors considéré comme étant un paramètre spécifique d'enfant.

12. Dispositif (100) selon la revendication 10 ou 11, incluant en outre un créateur supplémentaire, désigné ici comme un créateur d'ensemble parent (5), configuré pour créer l'ensemble parent.

13. Dispositif (100) selon l'une quelconque des revendications 10 à 12, dans lequel le modificateur (30) configuré pour modifier une valeur de paramètre de configuration de l'ensemble parent est configuré pour,
si le modificateur (30) détermine que, pour un ensemble enfant, le paramètre de configuration correspondant n'est pas un paramètre spécifique d'enfant, le fait d'amener la valeur du paramètre de configuration correspondant d'un ensemble enfant à être modifiée ; et
si le modificateur (30) détermine que, pour un ensemble enfant, le paramètre de configuration correspondant est un paramètre spécifique d'enfant et le modificateur (30) détermine, par obtention ou réception d'une entrée à partir d'un utilisateur, que le paramètre spécifique d'enfant doit être écrasé, le fait d'amener la valeur du paramètre de configuration correspondant de l'ensemble enfant à être modifiée.

14. Dispositif (100) selon l'une quelconque des revendications 10 à 13, dans lequel les valeurs de paramètre de configuration sont des valeurs relatives aux réglages de configuration de systèmes de laboratoire.

15. Dispositif (100) selon la revendication 14, dans lequel les valeurs de paramètre de configuration sont des valeurs relatives à au moins l'un de :
la manière dont les données sont traitées avant d'être entrées dans des dispositifs d'un laboratoire ;
la manière dont les données circulent à travers les dispositifs du laboratoire ;
la manière dont les données sont traitées après être sorties des dispositifs du laboratoire ;
la manière dont les dispositifs sont configurés dans le laboratoire ;
la manière dont les interfaces utilisateur sont affichées sur les ordinateurs dans le laboratoire ; et
les droits d'accès à certaines fonctions sur les ordinateurs du laboratoire.

16. Programme informatique incluant des instructions lisibles par ordinateur configurées, lorsqu'elles sont exécutées sur un ordinateur, pour amener l'ordinateur à réaliser le procédé selon l'une quelconque des revendications 1 à 9.
